Europäisches Patentamt

European Patent Office

Office européen des brevets

(11) Veröffentlichungsnummer: **0 356 632**
**A2**

## (12) EUROPÄISCHE PATENTANMELDUNG

(21) Anmeldenummer: **89110519.9**

(22) Anmeldetag: **10.06.89**

(51) Int. Cl.5 **A61C 19/04 , A61B 5/05**

(30) Priorität: **27.08.88 DE 3829082**

(43) Veröffentlichungstag der Anmeldung:
**07.03.90 Patentblatt 90/10**

(84) Benannte Vertragsstaaten:
**AT BE CH DE FR GB IT LI LU NL SE**

(71) Anmelder: **Wieland Edelmetalle KG**
**Schwenninger Strasse 13**
**D-7530 Pforzheim(DE)**

(72) Erfinder: **Kappert, Heinrich Friedrich, Prof. Dr.**
**Am Strässle 3**
**D-7806 March-Hugstetten(DE)**
Erfinder: **Gutekunst, Gerhard, Dr.**
**Rudolfstrasse 15**
**D-7500 Karlsruhe 1(DE)**

(74) Vertreter: **Schneck, Herbert, Dipl.-Phys., Dr. et al**
**Rau & Schneck Patentanwälte Königstrasse 2**
**D-8500 Nürnberg 1(DE)**

(54) **Vorrichtung zur Ermittlung der individuellen Empfindlichkeitsschwelle gegenüber galvanischen Spannungen in der Mundhöhle.**

(57) Es ist eine Vorrichtung zur Ermittlung der individuellen Empfindlichkeitsschwelle gegenüber galvanischen Spannungen in der Mundhöhle vorgesehen, um derartige Messungen als Entscheidungshilfe dafür heranziehen zu können, ob die aufgrund von Kontakten unterschiedlicher Metalle an Zähnen in der Mundhöhle auftretenden Spannungen Ursachen für Schmerzen oder Unwohlsein eines bestimmten Patienten sind. Eine derartige Vorrichtung zeichnet sich aus durch eine Einrichtung zum Erzeugen von Spannungsimpulsen, eine Einrichtung (16) zum definierten Verändern der Impulshöhe und ein Elektrodenpaar (9, 10) zum Anlegen der Spannungsimpulse in der Mundhöhle.

EP 0 356 632 A2

Die Erfindung richtet sich auf eine Vorrichtung zur Ermittlung der individuellen Empflindlichkeitsschwelle gegenüber galvanischen Spannungen in der Mundhöhle.

Derartige galvanische Spannungen treten auf, wenn an Zähnen Amalgamfüllungen angebracht oder sonstige metallische Überzüge in die Mundhöhle eingebracht werden und dabei zwei unterschiedliche Metalle in Kontakt kommen.

Das dauernde Vorhandensein derartiger galvanischer Spannungen kann bei den betroffenen Personen zu Unbehagen, aber auch zu ausgesprochenen Schmerzen führen. Man hat deshalb bereits die aufgrund einer bestimmten Füllung beispielsweise entstandenen Spannungen mit Hilfe von Spannungsmeßinstrumenten gemessen und, wenn diese Spannungen einen bestimmten Betrag überschreiten, eine vorhandene Amalgamfüllung durch eine Kunststoff- oder Edelmetallfüllung ersetzt.

Da ein derartiger Ersatz von an sich noch funktionsfähigen Füllungen durchaus aufwendig ist, sollte er vermieden werden, wenn dies durch das Schmerzempfinden des Patienten nicht zwingend geboten ist. Da die individuelle Reizempflindlichkeit verschiedener Personen ganz unterschiedlich ist, stellt es ein wenig geeignetes Kriterium dar, ungeachtet dieser spezifischen Reizempflindlichkeit jeweils oberhalb eines bestimmten Schwellwertes eine Behandlung vorzunehmen.

Demgegenüber wäre eine medizinisch sinnvolle und gezielte Behandlung dann möglich, wenn es gelingen würde, die spezifische Sensibilität einer bestimmten Person ebenso wie die sich an einem Zahnbereich ausbildende Spannung meßtechnisch zu erfassen und in Abhängigkeit von beiden Meßwerten zu entscheiden, ob tatsächlich eine bestimmte Füllung für eine Beeinträchtigung des Wohlbefindens verantwortlich ist und dementsprechend ein Austausch geboten erscheint.

Dementsprechend liegt der Erfindung die Aufgabe zugrunde, eine Vorrichtung zu schaffen, welche neben der an sich bekannten Bestimmung einer sich an einer bestimmten Metallkombination an einem Zahn oder Zahnbereich ausbildenden Spannung auch die Erfassung der individuellen Reizempflindlichkeit in praxisgerechter Weise ermöglicht.

Diese Aufgabe wird erfindungsgemäß gelöst durch eine Einrichtung zur Erzeugung von Spannungsimpulsen, eine Einrichtung zum definierten Verändern der Impulshöhe und ein Elektrodenpaar zum Anlegen der Spannungsimpulse in der Mundhöhle.

Durch eine derartige Vorrichtung wird es möglich, ausgehend von Spannungsimpulsen sehr niedriger Amplitude die Impulshöhe so lange zu verändern, bis der Patient eine typische Reaktion, wie z.B. Metallgeschmack, Elektrisierungswirkungen oder Zungenbrennen empfindet. Durch das Anlegen einer impulsförmigen Spannung wird das Ansprechverhalten des Patienten erheblich verbessert, da praktisch ein ständiger sensorischer Vergleich zwischen dem Zustand mit und ohne angelegter Spannung möglich ist.

In weiterer Ausgestaltung der Erfindung ist eine Starteinrichtung zum Auslösen der Einrichtung zum Verändern der Impulshöhe derart vorgesehen, daß die Impulshöhe mit fortschreitender Zeit stufenweise erhöht wird. Dementsprechend ist ein manuelles Verändern der Impulshöhe entbehrlich und es wird durch ein derartiges automatisches Hochfahren eine kontinuierliche, weitgehend lineare Veränderung ermöglicht. Dabei kann die Erhöhung so erfolgen, daß innerhalb der einzelnen "Stufen" eine bestimmte Impulsamplitude eine gewisse Zeit beibehalten wird, um dem Patienten auf diese Weise eine Reaktionszeit einzuräumen und dementsprechend eine besonders exakte Amplitudenbestimmung zu ermöglichen.

Günstigerweise ist eine Stopeinrichtung zum Anhalten der Einrichtung zum Verändern der Impulshöhe nach Erreichen der Reizschwelle und eine Meß- und Anzeigeeinrichtung zum Ermitteln und Anzeigen der dann anliegenden Impulshöhe vorgesehen. Hierdurch kann in einfacher Weise die beim Auftreten einer ersten Reaktion anliegende Impulshöhe bestimmt werden.

Mit Vorteil ist dabei vorgesehen, daß die Stopeinrichtung als ein über ein Kabel mit der übrigen Schaltungsanordnung verbundener, von dem Patienten bedienbarer Handschalter ausgebildet ist. Hierdurch entfällt die Notwendigkeit einer verbalen Mitteilung an den die Messung durchführenden Arzt, so daß eine größere Unmittelbarkeit der Reaktionen und damit auch eine höhere Genauigkeit der Messung gegeben ist. Gleichzeitig wird durch einen derartigen vom Patienten betätigbaren Handschalter dem Patienten das Gefühl gegeben, daß er ein gegebenenfalls auftretendes, unangenehmes Empfinden oder sogar einen Schmerz selbst sofort beenden kann, so daß auch ein im Hinblick hierauf denkbarer innerer Widerstand des Patienten gegen eine derartige Messung reduziert wird.

Die Meßeinrichtung ist vorteilhafterweise als hochomiges Digitalvoltmeter mit Haltefunktion ausgebildet. Die Hochomigkeit ist eine Voraussetzung dafür, daß die zu messende Spannung nicht durch das Meßgerät als solches beeinträchtigt wird, d.h. zusammenbricht. Die Haltefunktion des Meßinstruments ermöglicht es, den jeweils zuletzt anliegenden Meßwert in einem stationären Zustand abzulesen, d.h. es wird eine problemlose und auch genaue Ablesung ermöglicht. Um zu verhindern, daß die Elektroden als solche mit ein Potential ausbilden und damit das Meßergebnis verfälschen, ist vorgesehen, daß Edelmetall-Elektroden, insbesondere Platin- oder Goldelektroden verwendet werden.

Die Einrichtung zum Verändern der Impulshöhe kann mit einer Impulshöhe von ca. 0,1 Volt beginnen und diese in Schritten von 0,05 bis 0,1 Volt erhöhen. Grundsätzlich sind im Rahmen der Erfindung auch andere Abstufungen denkbar, wobei diese bevorzugt vorgeschlagene Abstufung den im Durchschnitt anzutreffenden natürlichen Gegebenheiten Rechnung trägt.

Dies gilt entsprechend auch für die vorzugsweise vorgesehene Impulsdauer von 0,5 bis 1 sec. und den Impulsabstand von 2 bis 3 sec.

Um eine erfindungsgemäße Vorrichtung alternativ auch in an sich bekannter Weise zur Messung der vorhandenen Spannung an einer bestimmten Metallkombination im Zahnbereich heranziehen zu können, ist eine Umschalteinrichtung zum Aktivieren der Einrichtung zum Erzeugen von Spannungsimpulsen und zur direkten Verbindung der Elektroden mit der Meß- bzw. Anzeigeeinrichtung vorgesehen.

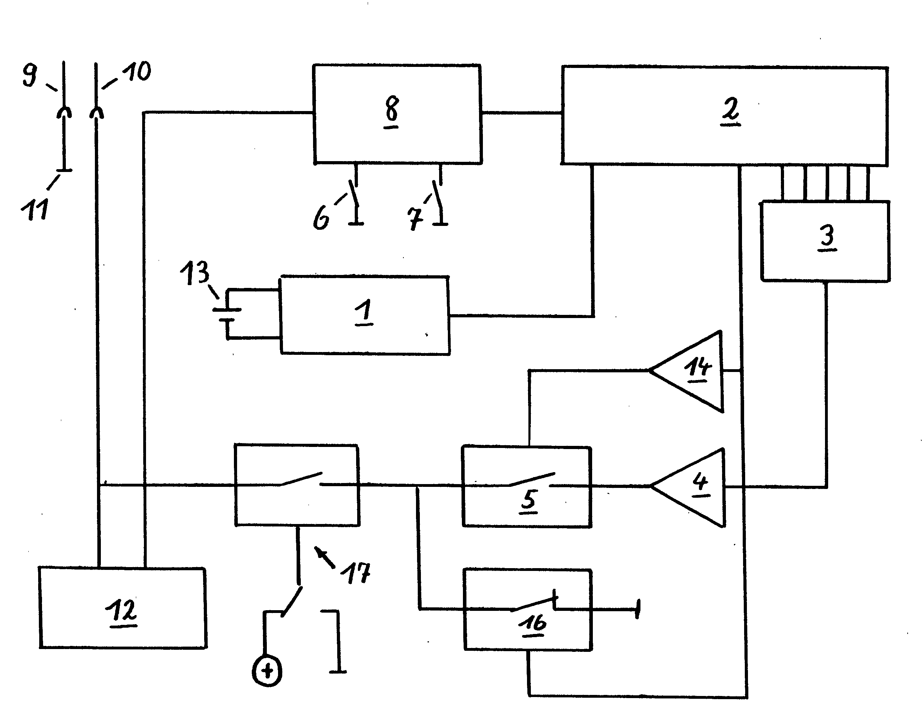

Nachfolgend wird ein bevorzugtes Ausführungsbeispiel einer erfindungsgemäßen Vorrichtung anhand der Zeichnung näher erläutert, welche eine schematische, blockschaltbildartige Darstellung der Schaltungsanordnung einer erfindungsgemäßen Vorrichtung zeigt.

Die in der Zeichnung dargestellte Schaltungsanordnung umfaßt ein stabilisiertes Spannungsversorgungsteil 1, einen Taktgeber 2, einen Digital-Analog-Wandler 3 mit nachgeschaltetem Verstärker 4 und einem Schalter 5. Weiterhin sind eine einen Startschalter 6 umfassende Starteinrichtung und eine einen Stopschalter 7 umfassende Stopeinrichtung, welche auf ein Flip-Flop 8 einwirken, vorgesehen.

Das Spannungsversorgungsteil 1 erzeugt aus einer Spannung von 3 Volt der Batterie 13 eine stabilisierte 9-Volt-Spannung. Beim Unterschreiten der Batteriespannung wird dies durch eine im einzelnen nicht dargestellte Anzeigeeinrichtung angezeigt.

Zwei Platin-Elektroden 9 bzw. 10 sind mit Erde 11 bzw. mit dem Eingang einer Meß- und Anzeigeeinrichtung 12 verbunden.

Bei der Bestimmung der spezifischen Sensibilität arbeitet die Schaltungsanordnung so, daß der Taktgeber 2 über eine im einzelnen nicht dargestellte Teilerschaltung einen Takt von z.B. 2,5 sec. für den am Digital-Analog-Wandler 3 angeschlossenen, im einzelnen nicht dargestellten Zähler erzeugt sowie die Schaltspannung für die analogen Schalter, wobei der Schalter 5 über den Verstärker 4 die Spannungsimpulse an die Elektrode 10 anlegt und über einen Verstärker 14 zum Öffnen und Schließen im Takt angesteuert wird.

Ebenfalls vom Taktgeber 2 angesteuert wird eine Einrichtung 16 zum stufenweisen Verändern der Impulshöhe.

Zur Bestimmung der Reizschwelle wird die Starteinrichtung 6 in Form eines Schalters vom Arzt betätigt, wobei die Amplitude der Spannungsimpulse beginnend z.B. mit 0,5 Volt bis auf maximal ca. 3 Volt erhöht wird.

Der Patient hält den als Handschalter ausgebildeten Schalter 7 der Stopeinrichtung in der Hand und betätigt diesen sobald er eine Reizwirkung wahrnimmt.

An der Meß- und Anzeigeeinrichtung wird dann der zuletzt anliegende Meßwert "gehalten" und kann bequem abgelesen werden. Bei einer Wiederholung der Messung oder bei einer Messung an einem anderen Patienten wird die Meßanordnung automatisch auf 0 zurückgesetzt, so daß Meßfehler vermieden werden.

Mittels einer Umschalteinrichtung 17 ist es möglich, die Anzeige- und Meßeinrichtung 12 direkt mit der Elektrode 10 unter Abschaltung der übrigen Einrichtung zur Erzeugung von Spannungsimpulsen zu verbinden, so daß in herkömmlicher Weise eine Spannungsmessung an einem bestimmten Zahn vorgenommen werden kann.

## Ansprüche

1. Vorrichtung zur Ermittlung der individuellen Empfindlichkeitsschwelle gegenüber galvanischen Spannungen in der Mundhöhle gekennzeichnet durch eine Einrichtung zum Erzeugen von Spannungsimpulsen, eine Einrichtung (16) zum definierten Verändern der Impulshöhe und ein Elektrodenpaar (9, 10) zum Anlegen der Spannungsimpulse in der Mundhöhle.

2. Vorrichtung nach Anspruch 1, gekennzeichnet durch eine Starteinrichtung 6 zum Auslösen der Einrichtung (16) zum Verändern der Impulshöhe derart, daß die Impulshöhe mit fortschreitender Zeit stufenweise erhöht wird.

3. Vorrichtung nach Anspruch 2, gekennzeichnet durch eine Stopeinrichtung (7) zum Anhalten der Einrichtung (16) zum Verändern der Impulshöhe nach Erreichen der Reizschwelle und eine Meß- und Anzeigeeinrichtung (12) zum Ermitteln und Anzeigen der dann anliegenden Impulshöhe.

4. Vorrichtung nach Anspruch 3, dadurch gekennzeichnet, daß die Stopeinrichtung (7) als ein über ein Kabel mit der übrigen Schaltungsanordnung verbundener, von dem Patienten bedienbarer Handschalter ausgebildet ist.

5. Vorrichtung nach Anspruch 3, dadurch gekennzeichnet, daß die Meßeinrichtung (12) ein hochohmiges Digitalvoltmeter mit Haltefunktion ist.

6. Vorrichtung nach Anspruch 1, dadurch gekennzeichnet, daß die Elektroden (9, 10) aus Edelmetall: hergestellt sind.

7. Vorrichtung nach Anspruch 2, dadurch gekennzeichnet, daß die Einrichtung (16) zum Verändern der Impulshöhe mit einer Impulshöhe von ca. 0,1 Volt beginnt und die Impulshöhe in Schritten von 0,05 bis 0,1 Volt erhöht.

8. Vorrichtung nach Anspruch 1, dadurch gekennzeichnet, daß die Impulsdauer 0,5 bis 1 sec. und der Impulsabstand 2 bis 3 sec. beträgt.

9. Vorrichtung nach Anspruch 1, gekennzeichnet durch eine Umschalteinrichtung (17) zum Deaktivieren der Einrichtung zum Erzeugen von Spannungsimpulsen und der direkten Verbindung der Elektroden (9, 10) mit der Meß- und Anzeigeeinrichtung (12).